# EUROPEAN PATENT SPECIFICATION

(11) **EP 4 247 248 B1**
(45) Date of publication and mention of the grant of the patent: **11.03.2026**
(21) Application number: 21819356.3
(22) Date of filing: 18.11.2021
(51) Int. Cl.: A61B 5/08, A61B 5/091, A61B 5/00

(54) **TECHNIQUES FOR EXTRACTING RESPIRATORY PARAMETERS FROM NOISY SHORT DURATION THORACIC IMPEDANCE MEASUREMENTS**
VERFAHREN ZUR EXTRAKTION VON ATEMPARAMETERN AUS RAUSCHBEHAFTETEN KURZFRISTIGEN THORAXIMPEDANZMESSUNGEN
TECHNIQUES D'EXTRACTION DE PARAMÈTRES RESPIRATOIRES À PARTIR DE MESURES D'IMPÉDANCE THORACIQUE DE COURTE DURÉE BRUITÉES

(30) Priority: 19.11.2020 US 202063115762 P
(43) Date of publication of application: 27.09.2023
(73) Proprietor: Analog Devices International Unlimited Company, Limerick (IE)
(72) Inventor: JAYARAMAN, Rajalakshmi, Chennai, 600100 (IN); GANESAN, Sriram, Bangalore, Karnataka 560048 (IN); BHATT, Nayan Sureshkumar, Surat Gujarat 395005 (IN); REYNOLDS, Abigail, Somerville, Massachusetts 02144 (US); SEO, Joohyun, Medford, Massachusetts 02155 (US); PAN, Guolin, Westwood, Massachusetts 02090 (US); AKL, Tony J., Bedford, Massachusetts 01730 (US); GOPINATHAN, Venugopal, Boston, Massachusetts 02114 (US)
(74) Representative: Yang, Shu
(86) International application number: PCT/EP2021/082219
(87) International publication number: WO 2022/106578

(56) References cited:
- US-A1- 2007 073 168
- US-A1- 2011 001 497
- US-A1- 2016 022 164
- US-A1- 2016 135 715
- US-A1- 2019 223 782

## Description

### RELATED APPLICATIONS

The present disclosure claims priority to U.S. Provisional Patent Application No. 63/115,762 entitled "TECHNIQUES FOR EXTRACTING RESPIRATORY PARAMETERS FROM NOISY SHORT DURATION THORACID IMPEDANCE MEASUREMENTS" and filed November 19, 2020.

### FIELD OF THE DISCLOSURE

This disclosure relates generally to techniques for detecting respiratory parameters from thoracic impedance measurements and, more particularly, to techniques for extracting such parameters from noisy short duration thoracic impedance measurements. US2019/223782 A1 discloses systems and methods for sensing respiration from a subject are discussed. An embodiment of a respiration monitoring system may include a respiration analyzer circuit to select a physiologic signal from a plurality of signals of different types indicative of respiration, such as between first and second physiologic signals that are respectively detected using first and second detection algorithms, and to compute one or more respiration parameters using the selected signal. The system may select or adjust a respiration detection algorithm for detecting the respiration parameters. The physiologic signal, or the respiration detection algorithm, may each be selected based on a signal characteristic or a patient condition. A cardiopulmonary event may be detected using the computed respiration parameter. The physiologic signal may include an impedance signal. The respiration analyzer circuit may select or adjust a respiration detection algorithm based on a signal characteristic, such as signal quality or computational complexity, or patient conditions. The respiratory rate may be determined using the timing of the zero-crossings. Acorrelator detects respiratory rate using an autocorrelation of a physiologic signal indicative of respiration.

### BRIEF DESCRIPTION OF THE DRAWINGS

To provide a more complete understanding of the present disclosure and features and advantages thereof, reference is made to the following description, taken in conjunction with the accompanying figures, wherein like reference numerals represent like parts, in which:
FIGURE 1 illustrates an example environment in which an illustrative system for deriving respiratory parameters from noisy short duration thoracic impedance measurements according to some embodiments of the disclosure;
FIGURE 2 is a block diagram illustrating exemplary functional components of the system of FIGURE 1, according to some embodiments of the disclosure;
FIGURE 3A is a flowchart illustrating operation of a method for extracting respiratory parameters from noisy short duration thoracic impedance measurements according to some embodiments of the disclosure;
FIGURE 3B is a flowchart illustrating operation of a method for extracting respiratory rate from noisy short duration thoracic impedance measurements according to some embodiments of the disclosure;
FIGUREs 4A and 4B respectively illustrate respiratory parameter extraction from a respiration modulated thoracic impedance signal using the autocorrelation based algorithm (FIGURE 4A) and the time domain based (zero-crossings) algorithm (FIGURE 4B) according to some embodiments of the disclosure;
FIGURE 5 is a flowchart illustrating a method for performing an impedance-specific signal quality check according to some embodiments of the disclosure;
FIGURE 6 are graphs illustrating the effects of noise and motion artifacts in a thoracic impedance signal according to some embodiments of the disclosure;
FIGURE 7 is a flowchart illustrating a method for performing an artifact detection signal quality check according to some embodiments of the disclosure;
FIGUREs 8A-8E illustrate a method for removing a detected artifact from a thoracic impedance signal according to some embodiments of the disclosure;
FIGURE 9 is a flowchart illustrating a method for evaluating the signal quality of the longest preprocessed good segment of a thoracic impedance signal according to some embodiments of the disclosure;
FIGURES 10A-10C are graphs collectively illustrating operation of the autocorrelation based algorithm for extracting RR from a thoracic impedance signal according to some embodiments of the disclosure;
FIGURES 11A and 11B collectively illustrate a flowchart showing operation of the autocorrelation based algorithm according to some embodiments of the disclosure;
FIGURE 12 is a graph illustrating the physiological significance of a thoracic impedance signal and its derivative as surrogates for thoracic volume (in liters) and flow (in liters/minute), respectively;
FIGURES 13A-13B collectively illustrate a flowchart showing operation of a time domain based zero-crossing algorithm according to some embodiments of the disclosure; and
FIGURES 14A-14F comprise graphs collectively illustrating operation of the time domain based zero-crossing algorithm shown in FIGURES 13A-13B.

### DESCRIPTION OF EXAMPLE EMBODIMENTS OF THE DISCLOSURE

Thoracic impedance measurements obtained using electrodes placed on a patient's thorax provide an indirect, non-invasive way to collect respiratory parameters of interest, due to the fact that the modulation of level of air in the lungs due to respiration will reflect in proportional modulation of the thoracic electrical impedance. However, such measurements are susceptible to extremely high levels of noisy artifacts, due to motion, cough, and/or improper skin-electrode contact, for example, making it challenging to extract parameters such as respiration rate (RR) and tidal volume (TV) from the measurements. Additionally, certain clinical conditions require extraction of events such as shallow breathing, apnea, and/or periodic or oscillatory breathing, for example, which are even more challenging to extract in the presence of the aforementioned artifacts. Embodiments described herein include two approaches for addressing the aforementioned issues, including a time domain based approach and an autocorrelation-based approach. Both approaches closely follow the physiological aspects of the respiratory cycle and maintain heuristic rules at a minimum, thus enabling extraction of most of these parameters from a single 60 second thoracic impedance measurement with error bounded within 2 breaths per minute (BPM), including quantization error.

Abnormal respiration activity of a person is an early indicator of respiratory, cardiac and/or neurological disease. Clinically, RR in breaths per minute is reported by counting number of the chest wall excursions during inhalation and exhalation. This method is often erroneous and depends on the skill level of the nurse. Clinical methods to extract TV (the volume of air inhaled and exhaled) involve breathing into a tube through the mouth with a nose clip, and thus are not usable for at-home monitoring.

As previously noted, thoracic impedance monitoring through electrodes placed on a person's thorax, may provide an indirect, non-invasive way to extract respiratory parameters such as RR and TV; however, accuracy of the technique is compromised by one or more of the presence of very low frequency baseline wander due to improper electrode contact to skin, high frequency physiological interferers such as cardiac activity, wide-band circuit noise and motion artifacts due to cough, hiccups, body movement, etc. Additionally, certain physiological conditions exhibit different signatures in the signal morphology, which makes it even harder to extract respiratory parameters with high confidence.

Traditional time domain-based approaches, such as peak detection/counting, and frequency domain-based approaches struggle to extract the parameters of interest from a thoracic impedance measurement signal (or simply thoracic impedance signal), due to the non-stationary nature of the signal itself, as well as the noise embedded in the signal.

Embodiments described herein offer a solution to these problems and provide techniques for reliably extracting respiratory parameters from a thoracic impedance signal through application of two different methodologies (time domain-based and autocorrelation based) in the presence of different physiological signal morphologies and artifact conditions. A novel approach to assess the signal quality of the thoracic impedance signal using the input signal, accelerometer data, and filtered noise is also presented.

A time domain-based approached is useful to report RR in case of low confidence RR estimates (not signal quality) from the autocorrelation based technique, as well as to estimate RR in cases of apnea and to calculate TV.

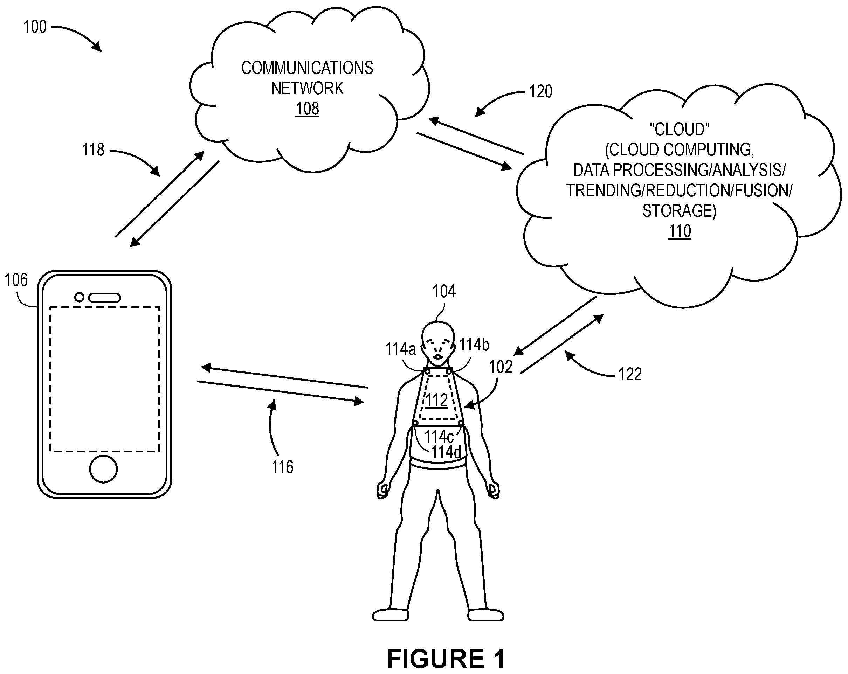

FIGURE 1 depicts an example environment 100 in which an illustrative embodiment of a system 102 for deriving and monitoring respiratory parameters, such as RR and TV, in human subjects using noisy short duration thoracic impedance measurements according to some embodiments of the disclosure. The monitoring may be performed in a continuous or periodic fashion. As shown in FIGURE 1, in accordance with one example embodiment, the system 102 includes a thoracic impedance measurement module 112 and a plurality of surface electrodes/sensors 114a-114d (e.g., four (4) surface electrodes/sensors, or any other suitable number of surface electrodes/sensors). For example, one or more of the surface electrodes can be implemented as solid-gel surface electrodes, or any other suitable surface electrodes. The system 102 can be configured as a generally triangular-shaped device, or any other suitably shaped device, operative to make contact with one or more of the torso, upper chest, and neck areas, or any other suitable parts or areas of the body, of a human subject 104 via at least the plurality of surface electrodes/sensors 114a-114d.

In various implementations, the system 102 can have a configuration that allows it to be implemented within a wearable vest-like structure, as multiple patch-like devices, or any other suitable structure or device(s). In one possible environment, such as the environment 100, the system 102 may be operative to engage in bidirectional communications over wireless communication paths 116 with a smartphone 106, which, in turn, may be operative to engage in bidirectional communications over wireless communication paths 118 with a communications network 108 (e.g., the Internet). Alternatively, a direct link to the cloud 110 may be provided without requiring a hop through a base station or cell phone. The smartphone 106 is further operative, via the communications network 108, to engage in bidirectional communications over wireless communication paths 120 with the cloud 110, which can include resources for cloud computing, data processing, data analysis, data trending, data reduction, data fusion, data storage, and other functions. The system 102 is further operative to engage in bidirectional communications over wireless communication paths 122 directly with the cloud 110.

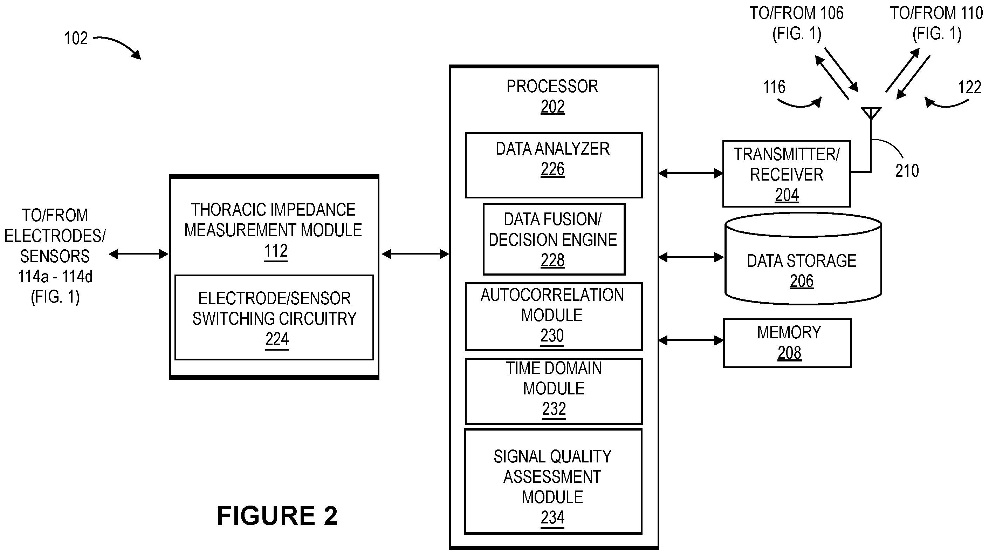

FIGURE 2 depicts an example block diagram of the system 102 for deriving and monitoring respiratory parameters, such as RR and TV, in human subjects using noisy short duration thoracic impedance measurements according to some embodiments of the disclosure. As shown in FIGURE 2, the system includes the thoracic impedance measurement module 112, a processor 202 and associated memory 208, a data storage 206 for storing thoracic impedance measurement data, and a transmitter/receiver 204. The transmitter/receiver 204 can be configured to perform Bluetooth communications, Wi-Fi communications, or any other suitable short-range communications for communicating with the smartphone 106 (FIGURE 1) over the wireless communication paths 116. The transmitter/receiver 204 can be further configured to perform cellular communications or any other suitable long-range communications for communicating with the cloud 110 (FIGURE 1) over the wireless communication paths 122. In certain embodiments, the thoracic impedance measurement module 112 may further include electrode/sensor connection switching circuitry 224 for switchably making connections with the plurality of surface electrodes/sensors 114a-114d shown in FIGURE 1.

The processor 202 can include a plurality of processing modules such as a data analyzer 226 and a data fusion/decision engine 228. The transmitter/receiver 204 can include at least one antenna 210 operative to transmit/receive wireless signals such as Bluetooth or Wi-Fi signals over the wireless communications paths 116 to/from the smartphone 106, which can be a Bluetooth or Wi-Fi-enabled smartphone or any other suitable smartphone. The antenna 210 is further operative to transmit/receive wireless signals such as cellular signals over the wireless communications paths 122 to/from the cloud 110.

The processor 202 can further include an autocorrelation module 230 and a time domain module 232 for respectively implementing an autocorrelation based technique and a time domain-based technique for deriving respiratory parameters from a thoracic impedance signal, as described herein. The processor 202 can further include a signal quality assessment module 234 for performing signal quality checks in connection with a thoracic impedance signal, as described herein.

The transmitter/receiver 204 can include at least one antenna 210 operative to transmit/receive wireless signals such as Bluetooth or Wi-Fi signals over the wireless communications paths 116 to/from the smartphone 106, which can be a Bluetooth or Wi-Fi-enabled smartphone or any other suitable smartphone. The antenna 210 is further operative to transmit/receive wireless signals such as cellular signals over the wireless communications paths 122 to/from the cloud 110.

The operation of the system 102 for deriving and monitoring respiratory parameters, such as RR and TV, in human subjects using noisy short duration thoracic impedance measurements according to some embodiments will be further understood with reference to the following illustrative example, as well as FIGURES 1 and 2. In this illustrative example, at fixed times each day (e.g., two times per day) or continuously for a predetermined number of days while the human subject 104 is in a supine or upright position, the human subject or a human assistant positions the system 102 configured as the generally triangular-shaped device (or any other suitably shaped device) such that it makes contact with one or more of the subject's torso and upper chest and neck areas (or any other suitable parts or areas of the body) via the plurality of surface electrodes/sensors 114a-114d.

Having positioned the system 102 in contact with the human subject's torso and/or upper chest and/or neck areas, the thoracic impedance measurement module 112 can be activated to gather, collect, sense, measure, or otherwise obtain thoracic impedance data from the human subject 104 and generate signals indicative thereof. In certain embodiments, the nature of the thoracic impedance data obtained using the illustrative method is noisy and of short duration.

The thoracic impedance measurement module 112 can perform thoracic impedance measurements using some or all of the plurality of surface electrodes 114a-114d that make contact with the skin of the human subject 104 on his or her torso, upper chest, and/or neck areas. In accordance with features of embodiments described herein, as will be described in greater detail hereinbelow, respiratory parameters, such as respiratory rate and tidal volume, may be derived from the noisy short duration thoracic impedance data from the thoracic impedance measurement module 112.

In some embodiments, the thoracic impedance data from the thoracic impedance measurement module 112 may be provided to the data analyzer 226 for at least partial data analysis, data trending, and/or data reduction. In one embodiment, the thoracic impedance measurement data in combination with other metadata, such as medical history, demographic information, and other testing modalities, can also be analyzed, trended, and/or reduced "in the cloud" and made available in cloud-based data storage 110 with pre-set alerts for use in various levels of clinical interventions with respect to respiratory parameters.

The data analyzer 226 may provide the at least partially analyzed thoracic impedance data to the data fusion/decision engine 228, which may effectively at least partially fuse or combine the thoracic impedance data with other sensing data, in accordance with one or more algorithms and/or decision criteria, for subsequent use in making one or more inferences about the human subject 104. The processor 202 may then provide the at least partially combined thoracic impedance and other sensing data to the transmitter/receiver 204, which may transmit the combined thoracic impedance and sensing data either directly over the wireless communication paths 122 to the cloud 110, or over the wireless communication paths 116 to the smartphone 106. Next, the smartphone 106 can transmit, via the communications network 108, the combined thoracic impedance and sensing data over the wireless communication paths 118, 120 to the cloud 110, where it can be further analyzed, trended, reduced, and/or fused. It will be recognized that, as described above, communications data may be communicated directly to the cloud 110 without involvement of a smartphone/cell phone or base station.

The resulting curated combined sensing data can then be remotely downloaded by hospital clinicians for risk scoring/stratification, monitoring and/or tracking purposes.

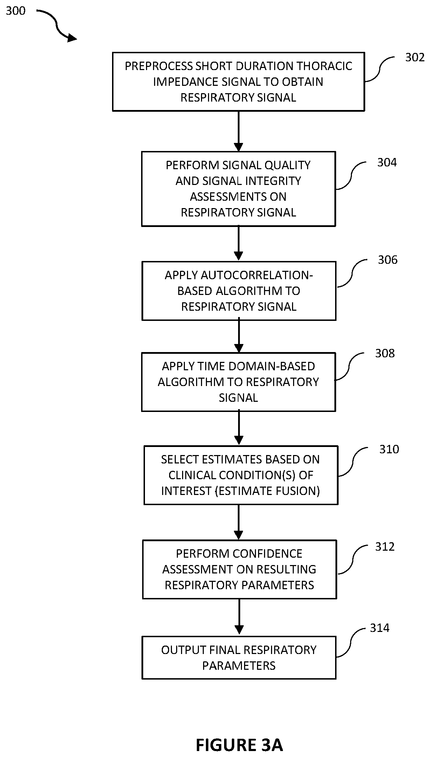

FIGURE 3A is a flowchart illustrating operation of a method 300 for extracting respiratory parameters from noisy short duration thoracic impedance measurements, or signals, according to some embodiments of the disclosure.

In step 302, a short duration (e.g., 60 second) thoracic impedance signal obtained using electrodes (e.g., electrodes 114 (FIGURE 1) placed on a human subject (e.g., human subject 104 (FIGURE 1)) is preprocessed to obtain a respiratory signal therefrom. In a particular embodiment, step 302 may be performed using a low pass filter with frequency cutoff (Fc) at 0.65Hz.

In step 304, signal quality and signal integrity assessments are performed on the respiratory signal (e.g., by the module 234 (FIGURE 2)). In a particular embodiment, the signal quality assessment may include computing certain thoracic impedance-specific metrics for the signal, such as electrode contact impedance and total body impedance, which are compared with established thresholds based on physiological limits. The signal integrity assessment may include checking a signature of the signal to detect and remove large artifacts or disturbances in the signal. Accelerometer data may be used to detect motion artifacts in the signal, which motion artifacts may also be removed in step 304.

As will be described in greater detail hereinbelow, certain embodiments of a method for extracting RR and TV from noisy short duration thoracic impedance signal, such as the method 300, exploit the fact that the thoracic impedance signal is a surrogate for lung volume of a human subject and the derivative of the thoracic impedance signal is a surrogate for air flow rate in and out of the human subject's lungs.

In general, autocorrelation algorithms derive the inherent periodicity event for non-stationary signals with noise. Referring again to FIGURE 3A, in step 306, the respiratory signal is processed using an autocorrelation based technique (e.g., implemented by the module 230 (FIGURE 2)). In particular, in step 306, the respiratory signal is autocorrelated to determine the second order average of the respiratory signal. In most autocorrelation based algorithms for extracting periodicity, the dominant peak, or local maxima, of the autocorrelated signal is alone considered; however, this is prone to errors due to large high/low frequency noise. In accordance with features of embodiments described herein, the autocorrelation based technique implemented in step 306 utilizes the entire autocorrelated respiratory signal to gain a better insight into the hidden periodicity and its variation in the signal. Accordingly, in the illustrated embodiment, in step 306, an expected value based on the time lags between the peaks in the autocorrelated signal is calculated to derive an estimated RR for the autocorrelation algorithm. This technique is well-suited for respiratory signals that have unusual morphology, periodic, oscillatory breathing patterns, and circuit noise.

In step 308, the respiratory signal is processed using a time domain based technique (e.g., implemented by module 232 (FIGURE 2)). In particular, and as will be described in greater detail hereinbelow, in step 308, the respiratory signal is divided into inhalation and exhalation cycles by computing the zero-crossings on the first order derivative of the respiratory signal. Heuristic rules based on physiological limitations, such as invalid RRs (e.g., more than 40 breaths per minute, less than 6 breaths per minute, etc.), and/or invalid inhalation to exhalation ratio (e.g., 1:4 or 4:1), are applied to identify valid breaths and eliminate invalid breaths. In accordance with embodiments described herein, the time domain based algorithm calculates RR by interval counting and calculates TV from the median of peak thoracic impedance values. The time domain based algorithm described herein is well-suited for respiratory signal with frequency and amplitude modulated breaths and apnea.

In step 310, estimates from the autocorrelation based algorithm and the time domain based algorithm may be selected based on certain signal signatures that represent certain clinical conditions. For example, in a case of apnea, which is absence of respiratory for few seconds, the number of inhalation and exhalations are best described by time domain based algorithm, whereas the autocorrelation based algorithm precisely specifies the rate at which the subject is breathing (i.e., the RR) before or after an apneic event. In contrast, in a case of oscillatory breath, the RR is specified by the autocorrelation based algorithm and oscillations in TV is specified by the time domain based "zero-crossing" algorithm.

In step 312, a confidence assessment may be performed on the estimates from the autocorrelation based algorithm and the time domain algorithm, as described hereinbelow.

In step 314, RR and TV estimates are selected and reported and/or recorded as desired.

It will be recognized that for thoracic impedance signals (e.g., those that have a significant amount of noise), a frequency domain method, such as the autocorrelation method, will be more useful in deriving RR from the thoracic impedance signal, whereas for other thoracic impedance signals (e.g., a thoracic impedance signal that is not particularly periodic), a time domain based method will be more useful in deriving RR from the thoracic impedance signal. Embodiments described herein leverage the relative advantages of both approaches, using both methods to derive RR and then selecting the one that is likely to be more accurate under the circumstances.

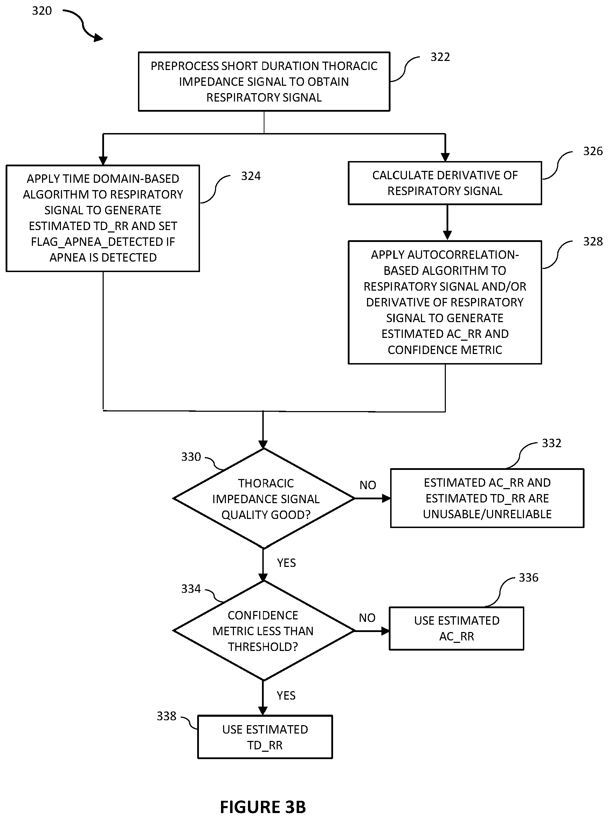

FIGURE 3B is a flowchart illustrating operation of a method 320 for detecting RR from noisy short duration thoracic impedance measurements, or signals, according to some embodiments of the disclosure.

In step 322, a short duration (e.g., 60 second) thoracic impedance signal obtained using electrodes (e.g., electrodes 114 (FIGURE 1) placed on a human subject (e.g., human subject 104 (FIGURE 1)) is preprocessed to obtain a respiratory signal therefrom. In a particular embodiment, step 322 may be performed using a low pass filter with frequency cut-off (Fc) at 0.65Hz. In some embodiments, the thoracic impedance signal may be filtered to a bandwidth of interest between 0.1Hz and 0.75Hz.

In step 324, In step 308, the respiratory signal is processed using a time domain based technique to generate an estimated time domain RR ("TD_RR"). Additionally, a FLAG_APNEA_DETECTED flag is set if apnea is detected in the respiratory signal during performance of the time-domain based technique.

In step 326, a derivative of the respiratory signal is calculated and in step 328, the respiratory signal and/or the derivative thereof are processed using the autocorrelation based technique to generate an estimated autocorrelation RR ("AC_RR"), as well as a confidence metric for the estimated AC_RR. In certain embodiments, the confidence metric (CM) is equal to the ratio of signal power (corresponding to breaths per minute (BPMs) within a range of ±5bpm of the estimated AC_RR) to the noise power (corresponding to BPMs outside the range of ±5bpm of the estimated AC_RR).

In step 328, a determination is made whether the quality of the thoracic impedance signal (as determined by one or more signal quality checks described hereinbelow) is good. If the quality of the thoracic impedance signal is not good, execution proceeds to step 332, in which a determination is made that there is no RR to report, as the signal is unreliable/unusable.

If it is determined in step 328 that the quality of the thoracic impedance signal is good, execution proceeds to step 334, in which a determination is made whether the confidence metric is less than a predetermined threshold (e.g., 1). If it is determined in step 334 that the confidence metric is less than the predetermined threshold, execution proceeds to step 336, in which the estimated TD_RR is output as the RR. If it is determined in step 334 that the confidence metric is not less than the predetermined threshold, execution proceeds to step 338, in which the estimated AC_RR is output as the RR.

In certain embodiments, the RR estimate (e.g., AC_RR or TD_RR) may be used to adjust the filtering used for TV extraction. For example, if RR is found to be 10bpm, the center frequency Fc and bandwidth of the low pass filter may be selected to be 10bpm+/-3bpm to improve TV extraction. It will be noted that TV information is one of the deciding factors for RR confidence metric (CM_RR) reporting. For example, a very low TV (possibly due to poor contact) or a very large TV (possibly due to contact impedance modulation) will both lower the confidence on RR reporting. Additionally, combining RR and TV information may provide important clinical insights. For example, minute ventilation is defined as the amount of air breathed per minute and is the product of RR and TV (e.g., 5-8 liters/minute, typically). Moreover, although TV is not directly detected in liters, by comparing the estimated TV with a baseline reading, possible hypoventilation/hyperventilation may be flagged if there is a significant decrease/increase in minute ventilation.

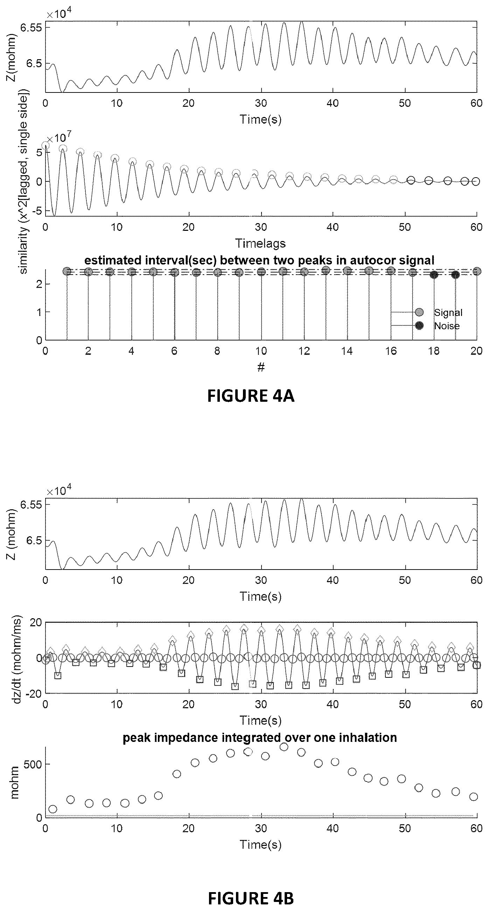

FIGURES 4A and 4B are graphs illustrating extraction of respiratory parameters from an oscillatory respiratory signal using the autocorrelation based algorithm (FIGURE 4A) and the time domain based (zero-crossings) algorithm (FIGURE 4B) according to some embodiments of the disclosure.

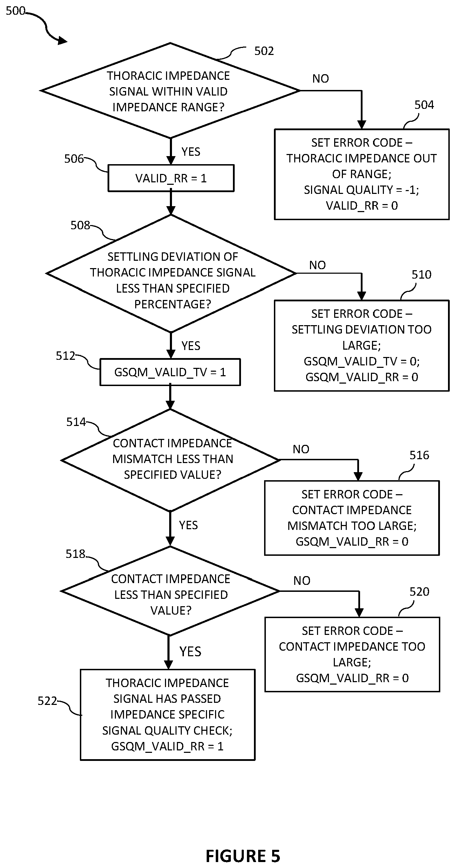

FIGURE 5 illustrates a flowchart of a method 500 for performing an impedance-specific signal quality check in accordance with embodiments described herein (e.g., as performed in step 304 (FIGURE 3A)). As shown in FIGURE 5, in step 502, a raw thoracic impedance ("TI") signal is checked to determine whether it is within a valid impedance range (e.g., greater than 30 ohms and less than 250 ohms). If it is determined that the thoracic impedance signal is not within the valid impedance range, execution proceeds to step 504, in which an error code is generated to indicate that the thoracic impedance signal is out of range and signal quality is rated a -1 ("no confidence"). Additionally in step 504, the value of a parameter valid_RR (which is a flag set to indicate whether the reported RR is valid) is set to 0 (i.e., reported RR is not valid). If it is determined in step 502 that the thoracic impedance signal is within the valid impedance range, execution proceeds to step 506, in which the value of valid_RR is set to 1 (i.e., reported RR is valid).

In step 508, a determination is made whether the settling deviation of the thoracic impedance signal is less than a specified percentage (e.g., 10%). As used herein, "settling deviation" refers to the change in thoracic impedance over the measurement time duration. For example, if thoracic impedance changes by more than 10%, the electrode contact is likely unstable. If it is determined that the settling deviation of the thoracic impedance signal is not less than the specified percentage, execution proceeds to step 510, in which an error code is generated to indicate that the thoracic impedance settling deviation is too large. Additionally in step 510, the value of a parameter gSQM_valid_TV is set to 0 and the value of a parameter gSQM_valid_RR is set to 0. If it is determined in step 508 that the settling deviation of the thoracic impedance signal is less than the specified percentage, execution proceeds to step 512, in which the value of gSQM_valid_TV is set to 1. It will be recognized that gSQM_valid_TV and gSQM_valid_RR are signal quality metrics for TV and RR, respectively, with a value of "1" indicating good signal quality and a value of "0" indicating poor signal quality.

In step 514, a determination is made whether a contact impedance mismatch is less than a particular value (e.g., 2000 ohms). If it is determined that the contact impedance mismatch is not less than the particular value, execution proceeds to step 516, in which an error code is generated to indicate that the contact impedance mismatch is too high. Additionally in step 516, the value of gSQM_valid_RR is set to 0. If it is determined in step 514 that the contact impedance mismatch is less than the particular value, execution proceeds to step 518.

In step 518, a determination is made whether the contact impedance is less than a particular value (e.g., 3000 ohms). If it is determined that the contact impedance is not less than the particular value, execution proceeds to step 520, in which an error code is generated to indicate that the contact impedance is too high. Additionally in step 520, the value of gSQM_valid_RR is set to 0. If it is determined in step 518 that the contact impedance is less than the particular value, execution proceeds to step 522.

In step 522, the signal is deemed to have passed the impedance-specific signal quality check and the value of gSQM_valid_RR is set to 1.

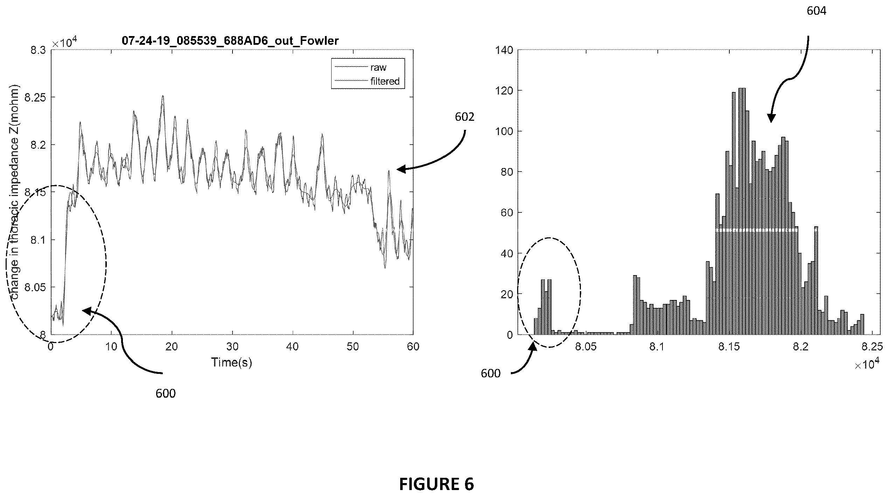

Referring now to FIGURE 6, it will be recognized that if there is a disturbance (e.g., an artifact) 600 in a thoracic impedance signal 602, the sample distribution 604 for the signal will likely tail in one direction because of large/very small numbers. In simpler terms, the presence of an artifact increases the signal deviation from the mean. To assess this, a coefficient of variation (CoV), which increases as the noise in the thoracic impedance signal increases, may be considered. It will be recognized that the standard deviation (std) of the thoracic impedance signal would also reflect this effect, but it is difficult to define an optimal threshold to accomplish this. In contrast, the CoV defines a ratio of noise to signal (std(signal)/mean(signal)). A CoV greater than 1 indicates the sample distribution is hyperexponential, whereas a CoV less than 0.4 indicates that the sample distribution is tailing in the opposite direction.

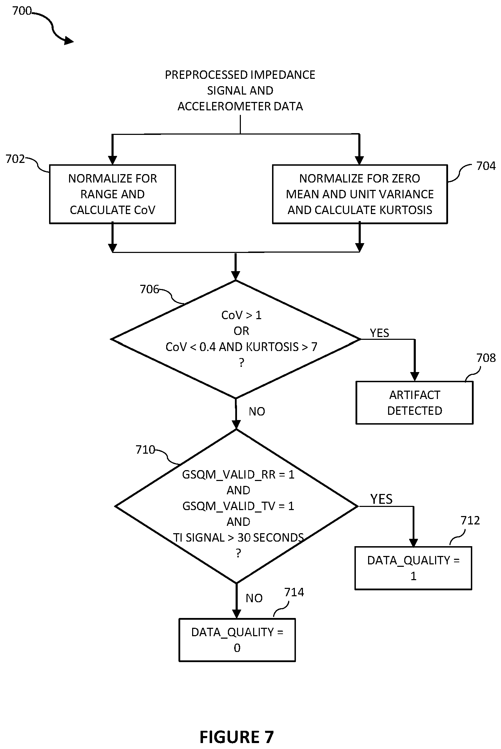

FIGURE 7 illustrates a flowchart of a method 700 for performing an artifact detection signal quality check in accordance with embodiments described herein (e.g., as performed in step 304 (FIGURE 3A)). Referring to FIGURE 7, in step 702, a preprocessed thoracic impedance signal and corresponding accelerometer data are normalized for range [0-1] to remove the effect of DC in the mean, and the CoV for the thoracic impedance signal is calculated. Substantially simultaneously, in step 704, the preprocessed thoracic impedance signal and accelerometer data are normalized for zero mean, unit variance, and kurtosis is calculated.

In step 706, a determination is made whether for either the thoracic impedance signal or the accelerometer data (1) CoV is greater than 1 or (2) CoV is less than 0.4 and kurtosis is greater than 7. If either of these conditions is true for either signal, in step 708, an artifact is detected. If neither of the conditions is true for either signal in step 706, execution proceeds to step 710.

In step 710, a determination is made whether gSQM_valid_RR = 1, gSQM_valid_TV = 1 (as determined in method 500 (FIGURE 5), and the length of the signal is greater than 30 seconds. If all of these conditions are true, execution proceeds to step 712, in which the signal is deemed to have high quality data confidence (data_quality = 1). If one or more of the conditions in step 710 is not true, execution proceeds to step 714, in which the signal is deemed to have low quality data confidence (data_quality = 0). As used herein, data_quality represents the final combined signal quality metric. For an artifact free signal of sufficient time duration (> 30sec), it is the logical AND of gSQM_valid_TV and gSQM_valid_RR, so it can be 1 or 0, depending on the SQMs of TV and RR. If the signal has artifacts or is of insufficient length, it is set to -1 (indicating that the signal is no good/unusable).

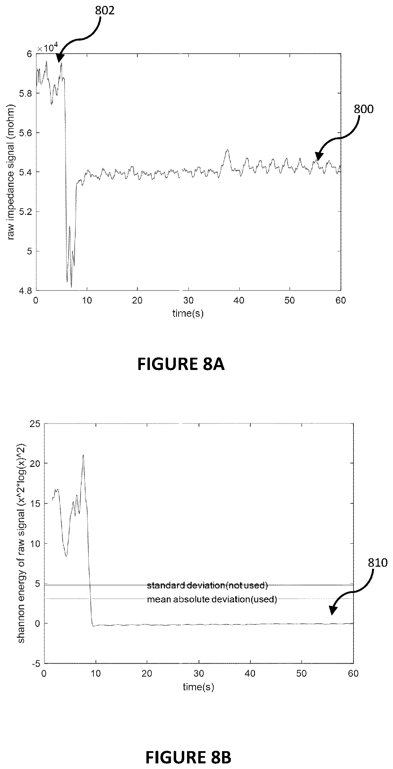

FIGURES 8A-8E illustrate a method for removing a detected artifact from a thoracic impedance signal in accordance with embodiments described herein to generate a signal from which RR and TV may be derived in accordance with embodiments described herein. FIGURE 8A illustrates a raw thoracic impedance signal 800 including an artifact 802. The raw thoracic impedance signal 800 is normalized for zero mean and unit variance. Additionally, a Shannon energy envelope is calculated for the thoracic impedance signal and a threshold is applied. It will be recognized that Shannon energy shows better differentiation than solely signal energy and it gives weight to a medium range artifact when compared to extremities. FIGURE 8B illustrates a waveform 810 representing the Shannon energy of the raw thoracic impedance signal 800 and the artifact 802.

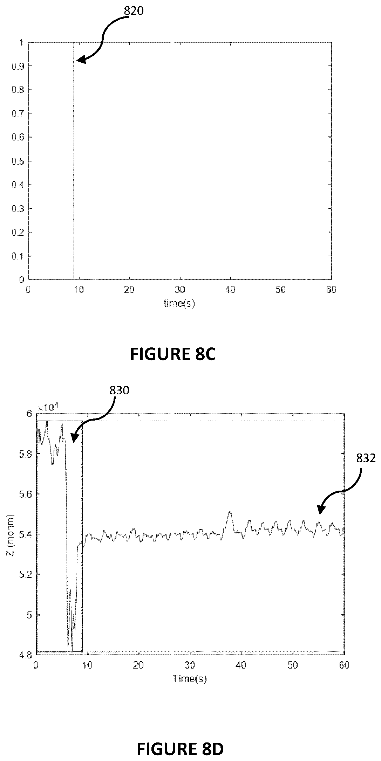

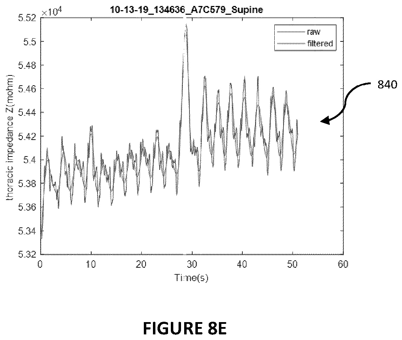

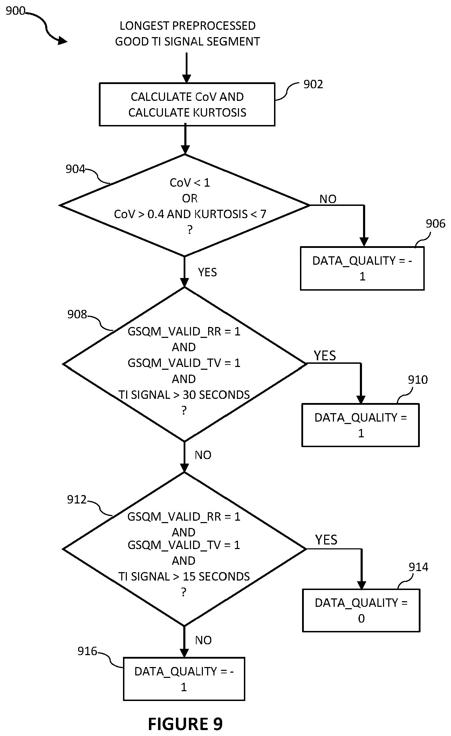

As shown in FIGURE 8C, a mask 820 is developed from the waveform 810 (FIGURE 8B) to identify a segment of the thoracic impedance signal that includes the artifact 802. Referring now to FIGURE 8D, the thoracic impedance signal is segmented into a bad segment 830 (which includes the artifact) and a good segment 832. The longest good segment, which in the embodiment illustrated in FIGURE 8D includes all of the good segment 832, is identified and preprocessed to create a longest preprocessed good segment, designated in FIGURE 8E by a reference numeral 840. The longest preprocessed good segment 840 is then used to derive RR and TV, as described herein. The signal quality of the longest preprocessed good segment 840 is evaluated as shown in FIGURE 9.

FIGURE 9 illustrates a method 900 for evaluating the signal quality of the longest preprocessed good segment, such as the segment 840 (FIGURE 8E). In step 902, a CoV and kurtosis for the segment is calculated. In step 904, a determination is made whether the CoV is less than 1 or the CoV is greater than 0.4 and the kurtosis is less than 7. If a negative determination is made in step 904, execution proceeds to step 906, in which a no quality confidence value is assigned to the segment and a data_quality parameter for the segment is set to -1.

If a positive determination is made in step 904, execution proceeds to step 908, in which a determination is made whether gSQM_valid_RR is equal to 1, gSQM_valid_TV is equal to one, and the signal length is less than 30 seconds. If all of the conditions specified in step 908 are met, execution proceeds to step 910, in which a high quality confidence value is assigned to the segment and the data_quality parameter is set to 1.

If one of the conditions specified in step 908 is not met, execution proceeds to step 912, in which a determination is made whether gSQM_valid_RR is equal to 1, gSQM_valid_TV is equal to one, and the signal length is less than 15 seconds. If all of the conditions specified in step 912 are met, execution proceeds to step 914, in which a low quality confidence value is assigned to the segment and the data_quality parameter is set to 0.

If one of the conditions specified in step 912 is not met, execution proceeds to step 916, in which a no quality confidence value is assigned to the segment and the data_quality parameter is set to -1.

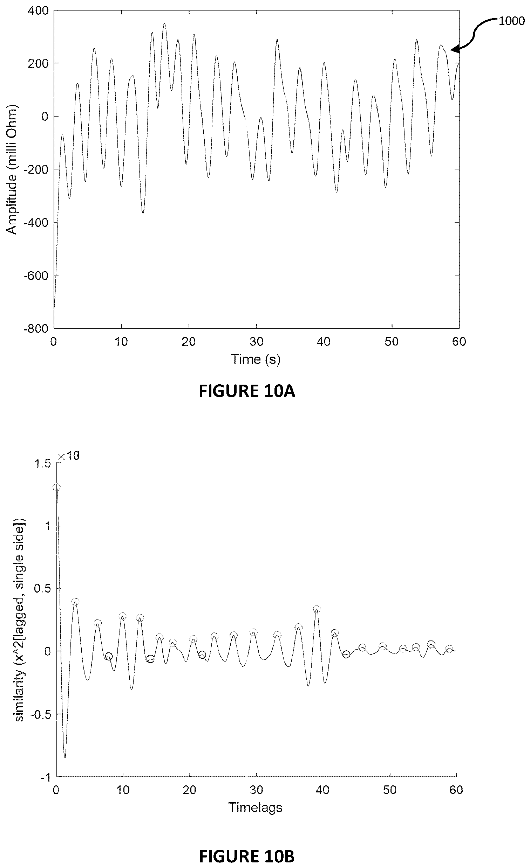

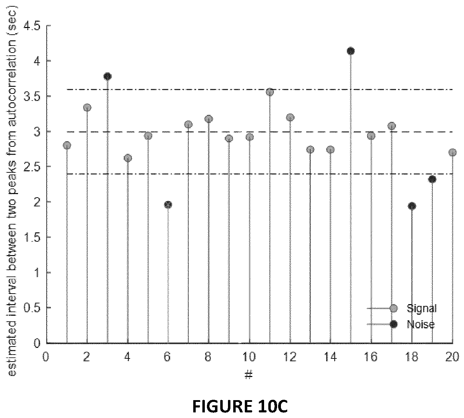

In accordance with details of particular embodiments, the autocorrelation based algorithm described herein derives the inherent periodicity of the respiratory signal (which need not be strictly periodic and/or stationary) without being affected by external noise. As will be described, use of the autocorrelation based algorithm to extract RR involves detrending the preprocessed signal to derive a trend stationary signal (zero mean), autocorrelation of the signal, and heuristic-based RR calculation from the autocorrelated signal. Additionally, a signal-to-noise ratio (SNR) and TV may be calculated from the autocorrelated signal. FIGURES 10A-10C illustrate operation of the autocorrelation based algorithm for extracting RR from a thoracic impedance signal 1000 (FIGURE 10A). As will be described in greater detail below, an expected value for RR is calculated (FIGURE 10B) and relative thresholding is used to qualify a peak as valid versus noise (FIGURE 10C).

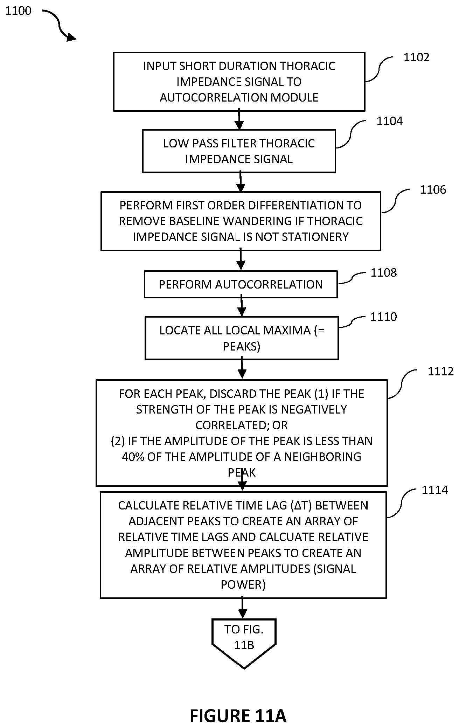

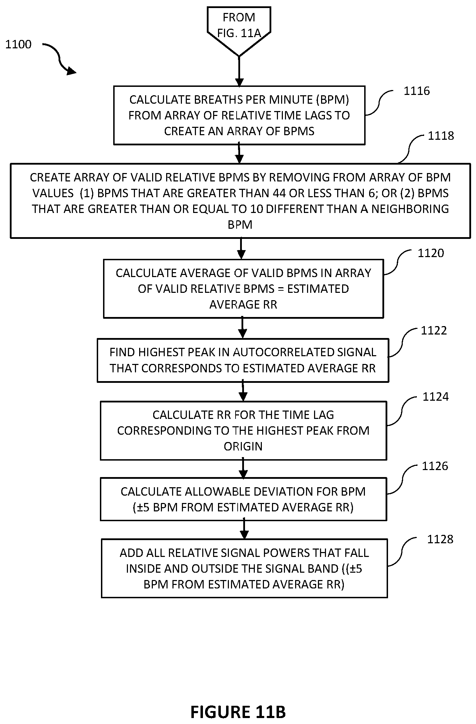

FIGURES 11A and 11B are a flowchart 1100 illustrating operation of the autocorrelation based module in accordance with embodiments described herein. In step 1102, 60 seconds of a thoracic impedance signal (e.g., a thoracic impedance signal segment) are input to the autocorrelation based module. In step 1104, the input thoracic impedance signal segment is low pass filtered to remove high frequency noise. In particular, the low pass filter may be a finite impulse response filter (FIR) having an Fc of 0.65Hz and length/3 number of taps.

In step 1106, first order differentiation is performed to remove baseline wandering (if the signal is not stationary) to produce a difference signal (Δamplitude/Δtime).

In step 1108, correlation is computed on the difference signal with a time lagged version of itself (lag of one sample) to produce an autocorrelated signal ((Δamplitude/Δtime)²).

In step 1110, all local maxima, or peaks, are identified in the autocorrelated signal.

In step 1112, a peak is discarded if the strength of the peak is negatively corelated and if the amplitude of the peak is less than 40% of the amplitude of neighboring peaks.

In step 1114, the relative amplitude and relative time lags between the peaks are calculated to produce an array of relative time lags equivalent to harmonic periods and an array of relative amplitudes, or signal powers.

In step 1116, an array of breaths per minute (BPMs) is calculated using the array of relative time lags (e.g., 60/ Δtime /sampling rate).

In step 1118, a BPM value may be eliminated from the array of BPMs calculated in step 1116 may be excluded from the array if (1) it is greater than 44 or less than 6 or (2) if the difference between the value and a neighboring BPM value is greater than or equal to 10. The result is an array of valid relative BPM values.

In step 1120, the average of the valid relative BPM values is calculated and deemed the estimated average RR.

In step 1122, the highest peak in the autocorrelated signal that corresponds to the estimated average RR is identified. This is the estimated dominant RR. The change in tidal impedance is equal to the square root of the highest signal peak.

In step 1124, the RR for the time lag corresponding to the highest peak from the origin is calculated and deemed the estimated dominant RR.

In step 1126, the allowable deviation for instantaneous BPM is calculated (e.g., the estimated average RR ±5).

In step 1128, all of the relative signal powers that fall inside (signal) and outside (noise) the signal band are summed to calculate the SNR.

The expected value of all the relative time lags represents the RR that is influenced by the harmonics of the highly periodic sequence in the thoracic impedance signal, increasing/decreasing frequency between cycles, low frequency artifacts, and uneven signal amplitudes (e.g., due to shallow breathing, apnea). At any time lag with a finite number of signals overlapped, only correlated data is represented as a peak and all the uncorrelated data are canceled. The algorithm does not entirely depend on the amplitude of the signal, so a large artifact has little effect. To identify a valid peak in the autocorrelated plot, relative threshold, rather than global threshold, is applied.

In accordance with features of embodiments described herein, a time domain-based approached is also provided and is useful to report RR in case of low confidence RR estimates (not signal quality) from the autocorrelation based technique, as well as to estimate RR in cases of apnea and to calculate TV.

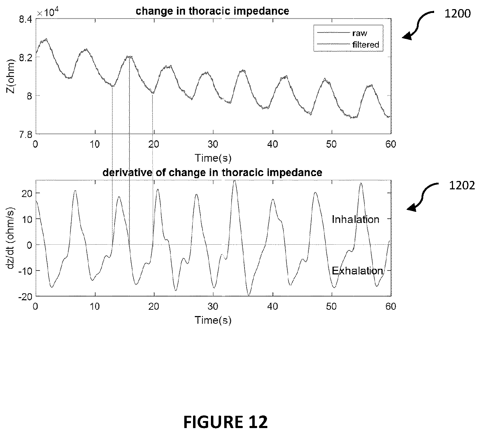

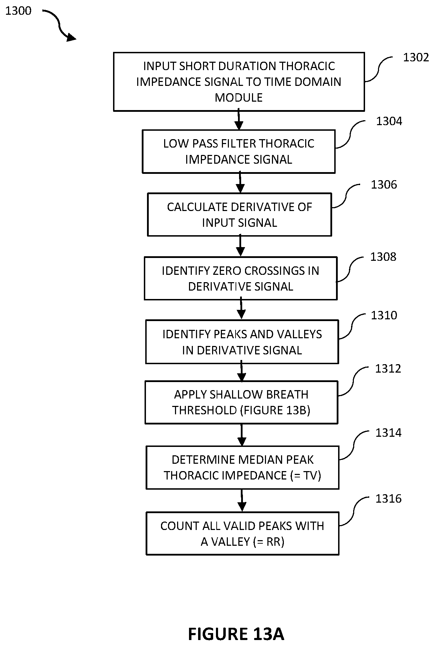

FIGURE 12 illustrates the physiological significance of the thoracic impedance signal and its derivative as surrogates for thoracic volume (in liters), as illustrated in a graph 1200, and flow (in liters/minute), as illustrated in a graph 1202, respectively. FIGURES 13A-13B illustrate operation of a method 1300 for implementing a time domain based zero-crossing in accordance with features of embodiments described herein. The time domain based algorithm is needed to report RR based on time domain counting in case of low confidence RR estimate from the autocorrelation based algorithm, to estimate RR in case of apnea, and to calculate TV, as will be described.

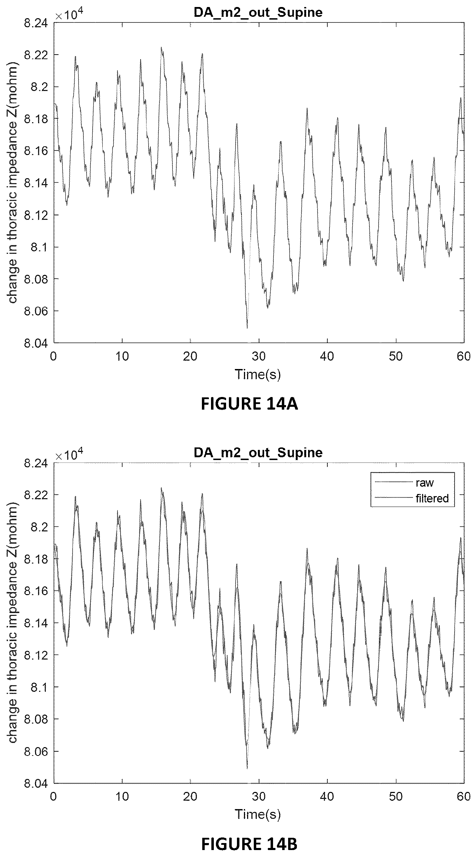

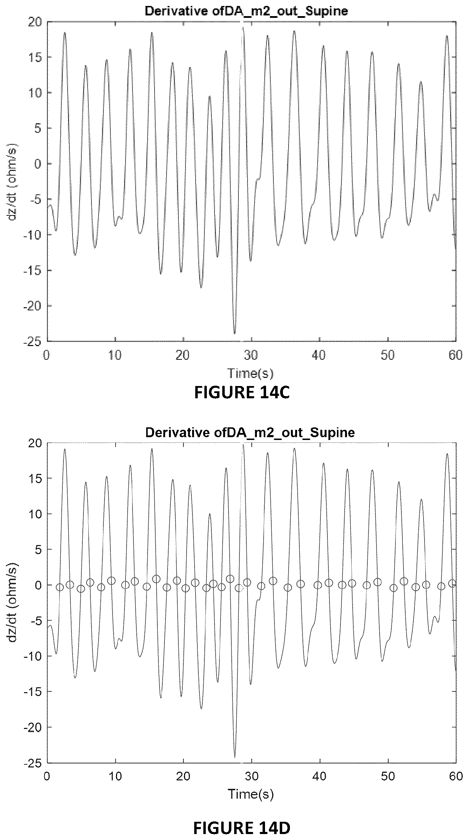

Referring to FIGURE 13A, in step 1302, a short duration thoracic impedance signal (as illustrated in FIGURE 14A) is input to the time domain module. In step 1304, the input signal is preprocessed by a low pass filter (e.g., at 0.65Hz) to create a filtered signal, illustrated in FIGURE 14B. In step 1306, a derivative of the preprocessed input signal is developed (FIGURE 14C) and in step 1308, zero-crossings in the derivative signal are identified (FIGURE 14D). In step 1310, peaks and valleys are identified in the derivative signal. Heuristic rules that are applied to identify a valid peak may include rejecting peaks that are less than a minimum threshold for a valid impedance peak (e.g., 5% of the highest peak, after artifact removal), rejecting inhalation peaks whose interval with the neighboring inhalation peak is less than 1.5s (40 bpm), and/or rejecting peaks whose peak inhalation and peak exhalation values vary by 90% (e.g., peak inhalation is 40 milliohm (mohm) and peak exhalation is 400 mohm).

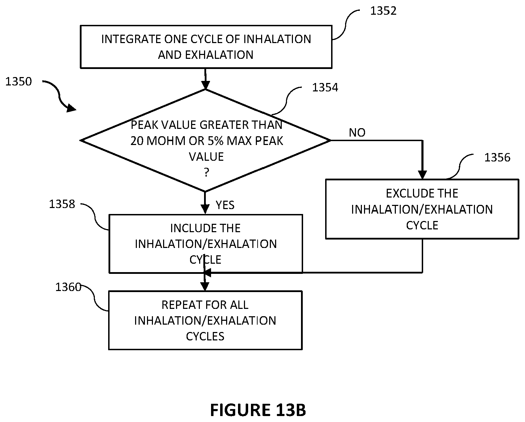

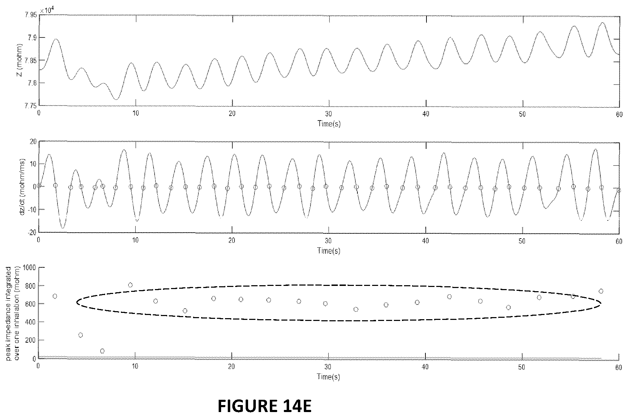

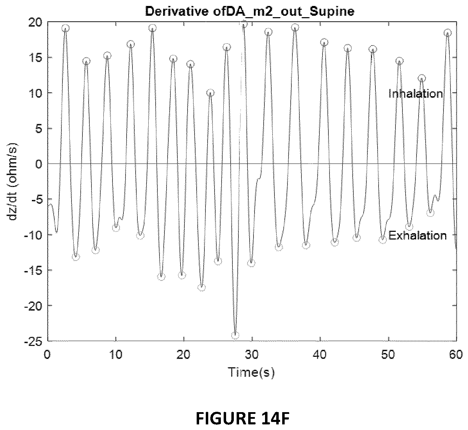

In step 1312, a shallow breath threshold (described in greater detail in FIGURE 13B) is applied. In step 1314, a median thoracic impedance value is calculated (FIGURE 14E) to produce a TV estimate. In step 1316, each valid peak with a valley is counted (FIGURE 14F) to produce an RR estimated using the time domain method ("RRt_estimate").

FIGURE 13B is a flowchart illustrating application of a shallow breath threshold method 1350 in accordance with embodiments described herein. As shown in FIGURE 13B, application of the shallow breath threshold includes integrating one cycle of inhalation and exhalation (step 1352) and then determining whether the peak value is greater than 20 (step 1mohm or 5% of maximum peak value (step 1354). If the peak value is not greater than 20 mohm or 5% of the maximum peak value, the inhalation/exhalation cycle is excluded from the count (step 1356). If the peak value is greater than 20 mohm or 5% of the maximum peak value, the inhalation/exhalation cycle is included in the count (step 1358). These foregoing steps are repeated for all inhalation/exhalation cycles (step 1360).

It should be noted that all of the specifications, dimensions, and relationships outlined herein (e.g., the number of elements, operations, steps, etc.) have only been offered for purposes of example and teaching only. The specifications apply only to one non-limiting example and, accordingly, they should be construed as such. In the foregoing description, exemplary embodiments have been described with reference to particular component arrangements. Various modifications and changes may be made to such embodiments without departing from the scope of the appended claims. The description and drawings are, accordingly, to be regarded in an illustrative rather than in a restrictive sense.

Note that with the numerous examples provided herein, interaction may be described in terms of two, three, four, or more electrical components. However, this has been done for purposes of clarity and example only. It should be appreciated that the system may be consolidated in any suitable manner. Along similar design alternatives, any of the illustrated components, modules, and elements of the FIGURES may be combined in various possible configurations, all of which are clearly within the broad scope of this Specification. In certain cases, it may be easier to describe one or more of the functionalities of a given set of flows by only referencing a limited number of electrical elements. It should be appreciated that the electrical circuits of the FIGURES and its teachings are readily scalable and may accommodate a large number of components, as well as more complicated/sophisticated arrangements and configurations. Accordingly, the examples provided should not limit the scope or inhibit the broad teachings of the electrical circuits as potentially applied to myriad other architectures.

It should also be noted that in this Specification, references to various features (e.g., elements, structures, modules, components, steps, operations, characteristics, etc.) included in "one embodiment", "exemplary embodiment", "an embodiment", "another embodiment", "some embodiments", "various embodiments", "other embodiments", "alternative embodiment", and the like are intended to mean that any such features are included in one or more embodiments of the present disclosure, but may or may not necessarily be combined in the same embodiments.

It should also be noted that the functions related to circuit architectures illustrate only some of the possible circuit architecture functions that may be executed by, or within, systems illustrated in the FIGURES. Some of these operations may be deleted or removed where appropriate, or these operations may be modified or changed considerably without departing from the scope of the present disclosure. In addition, the timing of these operations may be altered considerably. The preceding operational flows have been offered for purposes of example and discussion. Substantial flexibility is provided by embodiments described herein in that any suitable arrangements, chronologies, configurations, and timing mechanisms may be provided withoutdepa rtingf romtheteachings of the present disclosure.

Numerous other changes, substitutions, variations, alterations, and modifications may be ascertained to one skilled in the art and it is intended that the present disclosure encompass all such changes, substitutions, variations, alterations, and modifications as falling within the scope of the appended claims.

Note that all optional features of the device and system described above may also be implemented with respect to the method or process described herein and specifics in the examples may be used anywhere in one or more embodiments. The "means for" in these instances (above) may include (but is not limited to) using any suitable component discussed herein, along with any suitable software, circuitry, hub, computer code, logic, algorithms, hardware, controller, interface, link, bus, communication pathway, etc.

Note that with the example provided above, as well as numerous other examples provided herein, interaction may be described in terms of two, three, or four network elements. However, this has been done for purposes of clarity and example only. In certain cases, it may be easier to describe one or more of the functionalities of a given set of flows by only referencing a limited number of network elements. It should be appreciated that topologies illustrated in and described with reference to the accompanying FIGURES (and their teachings) are readily scalable and may accommodate a large number of components, as well as more complicated/sophisticated arrangements and configurations. Accordingly, the examples provided should not limit the scope or inhibit the broad teachings of the illustrated topologies as potentially applied to myriad other architectures.

It is also important to note that the steps in the preceding flow diagrams illustrate only some of the possible signaling scenarios and patterns that may be executed by, or within, communication systems shown in the FIGURES. Some of these steps may be deleted or removed where appropriate, or these steps may be modified or changed considerably without departing from the scope of the present disclosure. In addition, a number of these operations have been described as being executed concurrently with, or in parallel to, one or more additional operations. However, the timing of these operations may be altered considerably. The preceding operational flows have been offered for purposes of example and discussion. Substantial flexibility is provided by communication systems shown in the FIGURES in that any suitable arrangements, chronologies, configurations, and timing mechanisms may be provided without departing from the teachings of the present disclosure.

Although the present disclosure has been described in detail with reference to particular arrangements and configurations, these example configurations and arrangements may be changed significantly without departing from the scope of the present disclosure. For example, although the present disclosure has been described with reference to particular communication exchanges, embodiments described herein may be applicable to other architectures.

Numerous other changes, substitutions, variations, alterations, and modifications may be ascertained to one skilled in the art and it is intended that the present disclosure encompass all such changes, substitutions, variations, alterations, and modifications as falling within the scope of the appended claims. In order to assist the United States Patent and Trademark Office (USPTO) and, additionally, any readers of any patent issued on this application in interpreting the claims appended hereto, Applicant wishes to note that the Applicant: (a) does not intend any of the appended claims to invoke paragraph six (6) of 35 U.S.C. section 142 as it exists on the date of the filing hereof unless the words "means for" or "step for" are specifically used in the particular claims; and (b) does not intend, by any statement in the specification, to limit this disclosure in any way that is not otherwise reflected in the appended claims.

## Claims

1. A method (300) of determining a respiration rate, RR, of a human subject from a thoracic impedance, TI, measurement signal, the method comprising:
preprocessing (302) the TI measurement signal to generate a respiratory signal;
performing (304) a signal quality check on the respiratory signal;
executing (308) a time-domain based algorithm on at least a portion of the respiratory signal to determine an estimated time domain RR, TD_RR, comprising counting zero-crossings on a first order derivative of the respiratory signal to divide the respiratory signal into inhalation and exhalation cycles to calculate a RR, and applying heuristic rules to identify valid breath cycles and eliminate invalid breath cycles, including identifying invalid RRs and invalid inhalation to exhalation ratios, to determine the estimated TD_RR;
executing (306) an autocorrelation algorithm on the at least a portion of the respiration signal to determine an estimated autocorrelation RR, AC_RR, and a confidence metric for the estimated AC_RR, comprising autocorrelating the respiratory signal to determine an autocorrelated respiratory signal, and calculating an expected value based on time lags between peaks in the autocorrelated respiratory signal to derive an estimated RR;
selecting (312) one of the estimated TD_RR and the estimated AC_RR based on the confidence metric; and
outputting (312) the selected one of the estimated TD_RR and the estimated AC_RR as a final RR.

2. The method (300) of claim 1, wherein the selecting one of the estimated TD_RR and the estimated AC_RR based on the confidence metric comprises:
selecting the estimated AC_RR if the confidence metric is greater than or equal to a threshold value; and
selecting the estimated TD_RR if the confidence metric is less than the threshold value.

3. The method (300) of claim 1 or 2, further comprising, in response to a result of the signal quality check being poor, refraining from outputting the selected one of the estimated TD_RR and the estimated AC_RR as the final RR.

4. The method (300) of any of claims 1 to 3, wherein the preprocessing comprises filtering the TI measurement signal using a low pass filter.

5. The method (300) of any of claims 1 to 4, wherein the signal quality check comprises an impedance-specific signal quality check.

6. The method of claim 5, wherein the impedance specific signal quality check comprises checking at least one of electrode contact impedance and total body impedance with reference to thresholds based on physiological limits.

7. The method (300) of any of claims 1 to 6, wherein the signal quality check comprises identifying at least one signal artifact in the respiratory signal.

8. The method of claim 7, further comprising removing the at least one artifact from the respiratory signal to produce the at least a portion of the respiratory signal, the at least one artifact comprising noise and/or resulting from movement of the human subject.

9. The method (300) of any of claims 1 to 8, wherein the executing the autocorrelation algorithm on the at least a portion of the respiratory signal further comprises:
autocorrelating the at least a portion of the respiratory signal to determine an autocorrelated signal; and
calculating an expected value based on time lags between peaks in the autocorrelated signal to derive an estimated RR.

10. The method (300) of claim 9, wherein the confidence metric comprises a ratio of signal power to noise power for the autocorrelated signal.

11. The method (300) of any of claims 1 to 10, wherein the executing the time-domain zero-crossing algorithm on the at least a portion of the respiratory signal further comprises flagging an apnea condition in connection with the at least a portion of the respiratory signal and/or flagging a shallow breathing condition in connection with the at least a portion of the respiratory signal.

## Patentansprüche

1. Verfahren (300) zum Bestimmen einer Atemfrequenz, RR, eines menschlichen Subjekts aus einem Thoraximpedanz-, TI-, Messsignal, wobei das Verfahren Folgendes umfasst:
Vorverarbeiten (302) des TI-Messsignals, um ein Atmungssignal zu erzeugen;
Durchführen (304) einer Signalqualitätsprüfung am Atmungssignal;
Ausführen (308) eines auf Zeitbereich basierenden Algorithmus an mindestens einem Abschnitt des Atmungssignals, um eine geschätzte Zeitbereich-RR, TD_RR, zu bestimmen, umfassend das Zählen von Nulldurchgängen einer Ableitung erster Ordnung des Atmungssignals, um das Atmungssignal in Ein- und Ausatmungszyklen zu unterteilen, um eine RR zu berechnen, und Anwenden heuristischer Regeln, um gültige Atemzyklen zu identifizieren und ungültige Atemzyklen zu entfernen, einschließlich des Identifizierens ungültiger RR und ungültiger Verhältnisse von Einzu Ausatmung, um die geschätzte TD_RR zu bestimmen;
Ausführen (306) eines Autokorrelationsalgorithmus an dem mindestens einen Abschnitt des Atemsignals, um eine geschätzte Autokorrelations-RR, AC_RR, und eine Vertrauensmetrik für die geschätzte AC_RR zu bestimmen, umfassend das Autokorrelieren des Atmungssignals, um ein autokorreliertes Atmungssignal zu bestimmen, und Berechnen eines erwarteten Werts basierend auf Zeitspannen zwischen Spitzen im autokorrelierten Atmungssignal, um eine geschätzte RR abzuleiten;
Auswählen (312) eines der geschätzten TD_RR und der geschätzten AC_RR basierend auf der Vertrauensmetrik; und
Ausgeben (312) der einen Ausgewählten von der geschätzten TD_RR und der geschätzten AC_RR als finale RR.

2. Verfahren (300) nach Anspruch 1, wobei die eine Ausgewählte von der geschätzten TD_RR und der geschätzten AC_RR basierend auf der Vertrauensmetrik Folgendes umfasst:
Auswählen der geschätzten AC_RR, wenn die Vertrauensmetrik größer oder gleich groß wie ein Schwellenwert ist; und
Auswählen der geschätzten TD_RR, wenn die Vertrauensmetrik kleiner als der Schwellenwert ist.

3. Verfahren (300) nach Anspruch 1 oder 2, weiter umfassend, als Reaktion auf ein Ergebnis, dass die Signalqualitätsprüfung mangelhaft ist, Unterlassen des Ausgebens der einen Ausgewählten von der geschätzten TD_RR und der geschätzten AC_RR als die finale RR.

4. Verfahren (300) nach einem der Ansprüche 1 bis 3, wobei das Vorverarbeiten das Filtern des TI-Messsignals unter Verwendung eines Tiefpassfilters umfasst.

5. Verfahren (300) nach einem der Ansprüche 1 bis 4, wobei die Signalqualitätsprüfung eine impedanzspezifische Signalqualitätsprüfung umfasst.

6. Verfahren nach Anspruch 5, wobei die impedanzspezifische Signalqualitätsprüfung das Prüfen von mindestens einer von Elektrodenkontaktimpedanz und Gesamtimpedanz des Körpers in Bezug auf die Schwellenwerte basierend auf physiologischen Grenzwerten umfasst.

7. Verfahren (300) nach einem der Ansprüche 1 bis 6, wobei die Signalqualitätsprüfung das Identifizieren von mindestens einem Signalartefakt in dem Atmungssignal umfasst.

8. Verfahren nach Anspruch 7, weiter umfassend das Entfernen des mindestens einen Artefakts aus dem Atmungssignal, um den mindestens einen Abschnitt des Atmungssignals zu produzieren, wobei das mindestens eine Artefakt Rauschen umfasst und/oder aus einer Bewegung des menschlichen Subjekts resultiert.

9. Verfahren (300) nach einem der Ansprüche 1 bis 8, wobei das Ausführen des Autokorrelationsalgorithmus an dem mindestens einen Abschnitt des Atmungssignals weiter Folgendes umfasst:
Autokorrelieren des mindestens einen Abschnitts des Atmungssignals, um ein autokorreliertes Signal zu bestimmen; und
Berechnen eines erwarteten Werts basierend auf Zeitspannen zwischen Spitzen des autokorrelierten Signals, um eine geschätzte RR abzuleiten.

10. Verfahren (300) nach Anspruch 9, wobei die Vertrauensmetrik ein Verhältnis von Signalstärke zu Rauschleistung für das autokorrelierte Signal umfasst.

11. Verfahren (300) nach einem der Ansprüche 1 bis 10, wobei das Ausführen des Zeitbereichs-Nulldurchgangsalgorithmus an dem mindestens einen Abschnitt des Atmungssignals weiter das Kennzeichnen eines Apnoe-Zustands in Verbindung mit dem mindestens einen Abschnitt des Atmungssignals und/oder das Kennzeichnen eines Zustands flacher Atmung in Verbindung mit dem mindestens einen Abschnitt des Atmungssignals umfasst.

## Revendications

1. Procédé (300) de détermination d'une fréquence respiratoire, RR, d'un sujet humain à partir d'un signal de mesure d'impédance thoracique, TI, le procédé comprenant :
un prétraitement (302) du signal de mesure de TI pour générer un signal respiratoire ;
une mise en œuvre (304) d'un contrôle de qualité de signal sur le signal respiratoire ;
une exécution (308) d'un algorithme basé sur un domaine temporel sur au moins une partie du signal respiratoire pour déterminer un domaine temporel estimé RR, TD_RR, comprenant un comptage de passages par zéro sur une dérivée de premier ordre du signal respiratoire pour diviser le signal respiratoire en cycles d'inspiration et d'expiration pour calculer une RR, et une application de règles heuristiques pour identifier des cycles de respiration valides et éliminer des cycles de respiration invalides, incluant une identification de RR invalides et de rapports inspiration sur expiration invalides pour déterminer le TD_RR estimé ;
une exécution (306) d'un algorithme d'autocorrélation sur l'au moins une partie du signal de respiration pour déterminer une RR d'autocorrélation estimée, AC_RR, et une métrique de confiance pour l'AC_RR estimée, comprenant une autocorrélation du signal respiratoire pour déterminer un signal respiratoire autocorrélé, et un calcul d'une valeur attendue sur la base de décalages temporels entre des pics dans le signal respiratoire autocorrélé pour dériver une RR estimée ;
une sélection (312) d'un élément parmi le TD_RR estimé et l'AC_RR estimée sur la base de la métrique de confiance ; et
une émission (312) en sortie de l'élément sélectionné parmi le TD_RR estimé et l'AC_RR estimée en tant que RR finale.

2. Procédé (300) selon la revendication 1, dans lequel la sélection de l'élément parmi le TD_RR estimé et l'AC_RR estimée sur la base de la métrique de confiance comprend :
une sélection de l'AC_RR estimée si la métrique de confiance est supérieure ou égale à une valeur de seuil ; et
une sélection du TD_RR estimé si la métrique de confiance est inférieure à la valeur de seuil.

3. Procédé (300) selon la revendication 1 ou 2, comprenant en outre, en réponse à un résultat du contrôle de qualité de signal qui est médiocre, le fait de s'abstenir d'émettre en sortie l'élément sélectionné parmi le TD_RR estimé et l'AC_RR estimée en tant que RR finale.

4. Procédé (300) selon l'une quelconque des revendications 1 à 3, dans lequel le prétraitement comprend un filtrage du signal de mesure de TI à l'aide d'un filtre passebas.

5. Procédé (300) selon l'une quelconque des revendications 1 à 4, dans lequel le contrôle de qualité de signal comprend un contrôle de qualité de signal spécifique à l'impédance.

6. Procédé selon la revendication 5, dans lequel le contrôle de qualité de signal spécifique à l'impédance comprend un contrôle d'au moins une impédance parmi une impédance de contact d'électrode et une impédance de corps totale en référence à des seuils basés sur des limites physiologiques.

7. Procédé (300) selon l'une quelconque des revendications 1 à 6, dans lequel le contrôle de qualité de signal comprend une identification d'au moins un artefact de signal dans le signal respiratoire.

8. Procédé selon la revendication 7, comprenant en outre une élimination de l'au moins un artefact du signal respiratoire pour produire l'au moins une partie du signal respiratoire, l'au moins un artefact comprenant du bruit et/ou résultant d'un mouvement du sujet humain.

9. Procédé (300) selon l'une quelconque des revendications 1 à 8, dans lequel l'exécution de l'algorithme d'autocorrélation sur l'au moins une partie du signal respiratoire comprend en outre :
une autocorrélation de l'au moins une partie du signal respiratoire pour déterminer un signal autocorrélé ; et
un calcul d'une valeur attendue sur la base de décalages temporels entre des pics dans le signal autocorrélé pour dériver une RR estimée.

10. Procédé (300) selon la revendication 9, dans lequel la métrique de confiance comprend un rapport entre une puissance de signal et une puissance de bruit pour le signal autocorrélé.

11. Procédé (300) selon l'une quelconque des revendications 1 à 10, dans lequel l'exécution de l'algorithme de passage par zéro dans le domaine temporel sur l'au moins une partie du signal respiratoire comprend en outre un signalement d'un état d'apnée en relation avec l'au moins une partie du signal respiratoire et/ou un signalement d'un état de respiration superficielle en relation avec l'au moins une partie du signal respiratoire.
